# EUROPEAN PATENT SPECIFICATION

(11) **EP 4 014 858 B1**
(45) Date of publication and mention of the grant of the patent: **07.10.2026**
(21) Application number: 21215040.3
(22) Date of filing: 16.12.2021
(51) Int. Cl.: A61B 5/06, A61B 5/00, A61B 5/287, A61B 5/367

(54) **ACCURATE MEASUREMENT OF DISTAL END DIMENSION**
GENAUE MESSUNG DES DISTALEN ENDMASSES
MESURE PRÉCISE DE DIMENSION D'EXTRÉMITÉ DISTALE

(30) Priority: 17.12.2020 US 202017125879
(43) Date of publication of application: 22.06.2022
(73) Proprietor: Biosense Webster (Israel) Ltd, Yokneam, 2066717 (IL)
(72) Inventor: GOVARI, Assaf, Yokneam 2066717 (IL); GILNER, Vadim, Yokneam 2066717 (IL); BEECKLER, Christopher Thomas, Irvine 92618 (US)
(74) Representative: Carpmaels & Ransford LLP

(56) References cited:
- US-A1- 2018 344 202
- US-A1- 2020 206 461

## Description

### FIELD OF THE INVENTION

The present invention relates to medical systems, and in particular, but not exclusively, to catheter devices.

### BACKGROUND

A wide range of medical procedures involve placing probes, such as catheters, within a patient's body. Location sensing systems have been developed for tracking such probes. Magnetic location sensing is one of the methods known in the art. In magnetic location sensing, magnetic field generators are typically placed at known locations external to the patient. A magnetic field sensor within the distal end of the probe generates electrical signals in response to these magnetic fields, which are processed to determine the coordinate locations of the distal end of the probe. These methods and systems are described in U.S. Pat. Nos. 5,391,199, 6,690,963, 6,484,118, 6,239,724, 6,618,612 and 6,332,089, in PCT International Publication No. WO 1996/005768, and in U.S. Patent Application Publications Nos. 2002/0065455 and 2003/0120150 and 2004/0068178. Locations may also be tracked using impedance or current based systems.

US Patent Application Publication US 2020/0206461 describes a system including an expandable distal-end assembly, a proximal position sensor, a distal position sensor, and a processor. The expandable distal-end assembly is coupled to a distal end of a shaft for insertion into a cavity of an organ of a patient. The proximal and distal position sensors are located at a proximal end and a distal end of the distal-end assembly, respectively. The processor is configured to estimate a position and a longitudinal direction of the proximal sensor, and a position of the distal sensor, all in a coordinate system used by the processor. The processor is further configured to project the estimated position of the distal sensor on an axis defined by the estimated longitudinal direction, and calculate an elongation of the distal-end assembly by calculating a distance between the estimated position of the proximal sensor and the projected position of the distal sensor.

One medical procedure in which these types of probes or catheters have proved extremely useful is in the treatment of cardiac arrhythmias. Cardiac arrhythmias and atrial fibrillation in particular, persist as common and dangerous medical ailments, especially in the aging population.

Diagnosis and treatment of cardiac arrhythmias include mapping the electrical properties of heart tissue, especially the endocardium and the heart volume, and selectively ablating cardiac tissue by application of energy. Such ablation can cease or modify the propagation of unwanted electrical signals from one portion of the heart to another. The ablation process destroys the unwanted electrical pathways by formation of non-conducting lesions. Various energy delivery modalities have been disclosed for forming lesions, and include use of microwave, laser and more commonly, radiofrequency energies to create conduction blocks along the cardiac tissue wall. In a two-step procedure, mapping followed by ablation, electrical activity at points within the heart is typically sensed and measured by advancing a catheter containing one or more electrical sensors into the heart, and acquiring data at a multiplicity of points. These data are then utilized to select the endocardial target areas at which the ablation is to be performed.

Electrode catheters have been in common use in medical practice for many years. They are used to stimulate and map electrical activity in the heart and to ablate sites of aberrant electrical activity. In use, the electrode catheter is inserted into a major vein or artery, e.g., femoral vein, and then guided into the chamber of the heart of concern. A typical ablation procedure involves the insertion of a catheter having a one or more electrodes at its distal end into a heart chamber. A reference electrode may be provided, generally taped to the skin of the patient or by means of a second catheter that is positioned in or near the heart. RF (radio frequency) current is applied between the tip electrode(s) of the ablating catheter, and the reference electrode, flowing through the media between the electrodes it, i.e., blood and tissue0. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive.

### SUMMARY

The invention is defined in the claims.

There is provided in accordance with an embodiment of the present disclosure, a medical system including generator coils configured to generate respective magnetic fields having respective different frequencies in a region of a body part of a living subject, a catheter configured to be inserted into the body part of the living subject, and including a distal end, which includes magnetic coil sensors configured to output electrical signals as a function of detecting the respective magnetic fields, and including a first magnetic coil sensor having a first axis and a second magnetic coil sensor having a second axis, the magnetic coil sensors being disposed on the distal end with the first axis being substantially parallel with the second axis, and processing circuitry configured to receive the electrical signals from the magnetic coil sensors, select at least one of the magnetic fields having a magnetic field gradient, compute a difference between magnetic field magnitudes of the at least one selected magnetic field detected by the first magnetic coil sensor and the second magnetic coil sensor based on the received electrical signals, and compute a dimension of the distal end based on the magnetic field gradient and the difference between the magnetic field magnitudes.

Further in accordance with an embodiment of the present disclosure the computed dimension is the distance between the magnetic coil sensors.

Still further in accordance with an embodiment of the present disclosure the computed dimension is a dimension of a shape of the distal end of the catheter.

Additionally, in accordance with an embodiment of the present disclosure the processing circuitry is configured to compute the dimension of the distal end based onthe computed difference between the magnetic field magnitudes of the at least one selected magnetic field divided by the magnetic field gradient of the at least one selected magnetic field.

Moreover, in accordance with an embodiment of the present disclosure the at least one selected magnetic field includes one of the magnetic fields having a highest magnetic field gradient of the magnetic fields, and the processing circuitry is configured to compute the dimension of the distal end based on the computed difference between the magnetic field magnitudes of the at least one selected magnetic field and the highest magnetic field gradient.

Further in accordance with an embodiment of the present disclosure the catheter has a longitudinal axis, and the distal end of the catheter includes an expandable distal end assembly, the magnetic field sensors being configured to move with respect to each other along the longitudinal axis of the catheter as the expandable distal end assembly is expanded and collapsed, when the expandable distal end assembly is collapsed the distance between the magnetic coil sensors increases, and when the expandable distal end assembly is deployed the distance between the magnetic coil sensors decreases.

Still further in accordance with an embodiment of the present disclosure the first axis, second axis, and the longitudinal axis are substantially coaxial.

Additionally, in accordance with an embodiment of the present disclosure the expandable distal end assembly is a basket distal end assembly including a plurality of flexible strips and electrodes disposed on the flexible strips.

Moreover, in accordance with an embodiment of the present disclosure, the system includes a display, and wherein the processing circuitry is configured to find a shape of the distal end assembly based onat least the computed dimension, and render to the display a representation of the distal end assembly based on the found shape of the distal end assembly.

Further in accordance with an embodiment of the present disclosure the computed dimension is the distance between the magnetic coil sensors.

Still further in accordance with an embodiment of the present disclosure the processing circuitry is configured to compute a relative orientation between the first axis of the first magnetic coil sensor and the second axis of the second magnetic coil sensor, and estimate a shape of the distal end assembly based onthe computed relative orientation.
having a highest magnetic field gradient of the magnetic fields, and the computing the dimension includes computing the dimension of the distal end based on the computed difference between the magnetic field magnitudes of the at least one selected magnetic field and the highest magnetic field gradient.

Additionally, in accordance with an embodiment of the present disclosure, the method includes moving the magnetic field sensors with respect to each other along a longitudinal axis of the catheter as an expandable distal end assembly of the catheter is expanded and collapsed.

Moreover, in accordance with an embodiment of the present disclosure the first axis, second axis, and the longitudinal axis are substantially coaxial.

Further in accordance with an embodiment of the present disclosure, the method includes finding a shape of the distal end assembly based on at least the computed dimension, and rendering to a display a representation of the distal end assembly based on the found shape of the distal end assembly.

Still further in accordance with an embodiment of the present disclosure the computed dimension is the distance between the magnetic coil sensors.

Additionally, in accordance with an embodiment of the present disclosure, the method includes computing a relative orientation between the first axis of the first magnetic coil sensor and the second axis of the second magnetic coil sensor, and estimating a shape of the distal end assembly based on the computed relative orientation.

### BRIEF DESCRIPTION OF THE DRAWINGS

The present invention will be understood from the following detailed description, taken in conjunction with the drawings in which:
Fig. 1 is a schematic, pictorial illustration of a system for electro-anatomical mapping comprising a catheter, in accordance with an embodiment of the present invention;
Fig. 2A is a schematic view of a distal end of a basket catheter in a collapsed formation;
Fig. 2B is a schematic view of the distal end of the basket catheter of Fig. 2A in a deployed formation; and
Fig. 3 is a flowchart including steps in a method of operation of the system of Fig. 1.

### DESCRIPTION OF EXAMPLE EMBODIMENTS

### OVERVIEW

The Carto^{®}3 system (produced by Biosense Webster, Inc., Irvine, California) applies Advanced Catheter Location (ACL) hybrid position-tracking technology. In ACL technology, distribution of measured currents associated with probe electrodes on a catheter are correlated with a current-to-position matrix (CPM), which maps the current distribution to a position of the catheter that was previously acquired from magnetic location-calibrated position signals. The ACL technology enables locating and visualizing a catheter (even a catheter which does not have a magnetic field sensor), but only in the volume(s) where the CPM has been computed, using a catheter with a magnetic coil sensor. A prerequisite for building the CPM is to insert a magnetic-field sensor-equipped catheter into a body and move the catheter in a volume of the body, in order to compute the CPM for that volume.

Additionally, ACL technology may be used to track a basket catheter which has electrodes on the basket. However, ACL technology, which measures currents or impedances, may not provide high enough accuracy in some situations.

One solution is to use signals from magnetic sensors disposed on a catheter to compute the elongation of an expandable distal end assembly (such as a basket distal end assembly or a balloon distal end assembly) based on a distance between the magnetic sensors. The magnetic sensors can be placed on the catheter in such a manner that the distance between the sensors provides an indication of the elongation, and therefore the shape, of the distal end assembly. Magnetic sensors generally provide a more accurate position than using ACL. Nevertheless, the locations measured by the magnetic sensors are subject to errors, of the order of millimeters (e.g., 2 or 3 mm) and in some applications even these errors may be too large. For example, for a small basket catheter the distance between the magnetic sensors may change by about 10 or 15 millimeters between the basket being collapsed and the basket being deployed. Therefore, an error of 3 mm may be considered a large error. Errors may be reduced by using a Dual-Axis Sensor (DAS) or a Triple-Axis Sensor (TAS), which generally provide more accurate position measurements. However, in many applications, the catheter may not be able to accommodate two DASs or TASs or even one DAS or TAS. Details of magnetic location sensing are provided in commonly owned US Patent Nos. 5,391,199; 5,443,489, 5,558,091; 6,172,499; 6,690963; 6,788,967; and 6,892,091.

Embodiments of the present invention provide a system which accurately compute a dimension of a distal end (e.g., an expandable distal end assembly such as a basket or balloon distal end assembly) of a catheter using magnetic-based tracking technology based on two magnetic coil sensors and magnetic field generators that generate respective alternating magnetic fields (of different frequencies) for detection by the sensors. The magnetic fields detected by the sensors are indicative of the position of the sensors within a given coordinate space.

The accuracy of the computation is based on two factors including the positioning of the two magnetic coil sensors and an accurate error-canceling computational method.

The magnetic coil sensors are placed along a longitudinal axis of the distal end of the catheter so that the axes of the two sensors are substantially parallel, and in some embodiments the two sensors are placed to be substantially coaxial with the longitudinal axis. In this way, both sensors sense the different alternating magnetic fields in a similar way (e.g., with respect to magnetic field gradients) so that in the computation described in more detail below one of the alternating magnetic fields may be used for both sensors and error-canceling between the two sensors may take effect. The term "substantially parallel", as used in the specification and claims, is defined as parallel within a tolerance of 10 degrees. However, the closer the axes of the two sensors are to being exactly parallel, the computations performed based on the output of the sensors will be more accurate. The term "substantially coaxial", as used in the specification and claims, is defined as the axes of the sensors being within 10 degrees of the longitudinal axis and the region between the windings of the sensors intersecting the longitudinal axis.

The error-canceling computation method includes computing respective magnetic field gradients of the respective magnetic fields (in a direction parallel to the axes of the sensors) which is detected at the distal end (e.g., at one or more of the sensors). The term "magnetic field gradient", as used in the specification and claims, is defined as the change of a magnetic field over distance in a particular direction. In some embodiments, an approximate position of one or more of the magnetic field sensors may be computed using any suitable method and then based on a known function of the different magnetic fields over three-dimensional (3D) space, the magnetic field gradients at the distal end (in a direction parallel to the axes of the sensors) may be found for each of the magnetic fields.

One of the magnetic fields is selected (e.g., the magnetic field having the highest magnetic field gradient). In some embodiments, a subset of the magnetic fields is selected (e.g., having the highest magnetic field gradients) and an average magnetic field gradient of the selected magnetic fields is computed.

A difference between magnetic field magnitudes of the selected magnetic field detected by the sensors is computed. When a subset of magnetic fields is selected, an average difference between magnetic field magnitudes of the selected magnetic fields detected by the sensors is computed.

The distance between the sensors may then be computed based on the (average) magnetic field gradient of the selected magnetic field(s) and the (average) difference between the magnetic field magnitudes of the selected magnetic field(s). In some embodiments, the distance may be computed based on dividing the (average) difference between the magnetic field magnitudes of the selected magnetic field(s) by the (average) magnetic field gradient of the selected magnetic field(s). Another dimension of the distal end assembly may be computed from the computed distance between the sensors. The distance and/or the dimension may then be used to find a shape of the distal end assembly so that a representation of the distal end assembly may be rendered to a display.

### SYSTEM DESCRIPTION

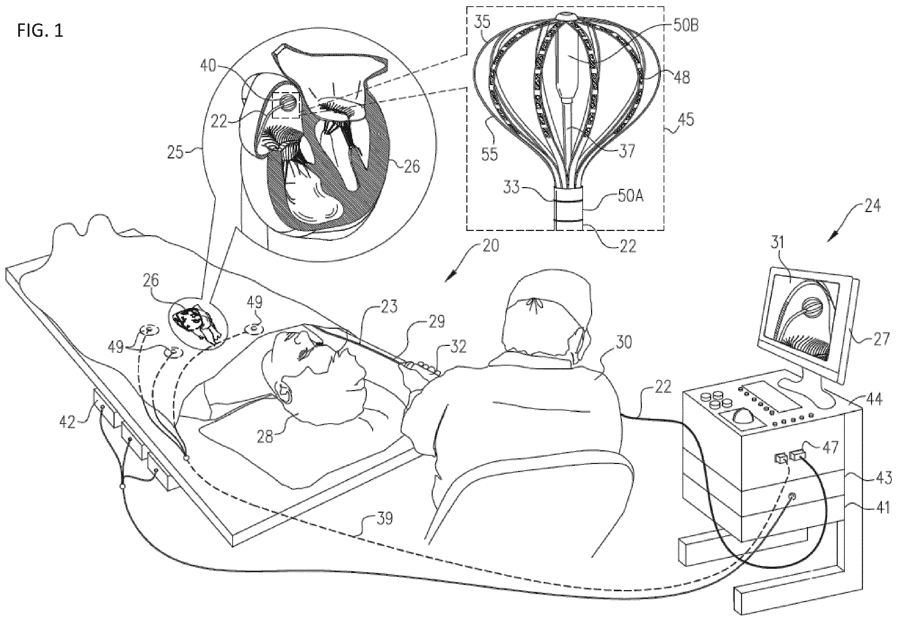

Reference is now made to Fig. 1, which is a schematic, pictorial illustration of a catheter tracking system 20, in accordance with an embodiment of the present invention. The system 20 includes a catheter 40 configured to be inserted into a body part of a living subject (e.g., a patient 28). A physician 30 navigates the catheter 40 (for example, a basket catheter produced by Biosense Webster, Inc. of Irvine, CA, USA), seen in detail in inset 45, to a target location in a heart 26 of the patient 28, by manipulating a deflectable segment of an insertion tube 22 of the catheter 40, using a manipulator 32 near a proximal end 29 of the insertion tube 22, and/or deflection from a sheath 23. In the pictured embodiment, physician 30 uses catheter 40 to perform electro-anatomical mapping of a cardiac chamber.

The catheter 40 includes a distal end 33. The distal end 33 of the catheter 40 includes an assembly 35 (e.g., a basket assembly as shown in Fig. 1 or a balloon assembly) on which multiple electrodes 48 (only some labeled for the sake of simplicity) are disposed. The assembly 35 is disposed distally to the insertion tube 22 and may be connected to the insertion tube 22 via a coupling member of the insertion tube 22 at the distal end 33. The coupling member of the insertion tube 22 may be formed as an integral part of the rest of the insertion tube 22 or as a separate element which connects with the rest of the insertion tube 22.

The assembly 35 further comprises multiple flexible strips 55 (only two labeled for the sake of simplicity), to each of which are coupled the electrodes 48. The assembly 35 may include any suitable number of electrodes 48. In some embodiments, the assembly 35 may include ten flexible strips 55 and 120 electrodes, with twelve electrodes disposed on each flexible strip 55.

The catheter 40 includes a pusher 37. The pusher 37 is typically a tube that is disposed in a lumen of the insertion tube 22 and spans from the proximal end 29 to the distal end 33 of the insertion tube 22. A distal end of the pusher 37 is connected to first ends of the flexible strips 55, typically via a coupling member of the pusher 37. The coupling member of the pusher 37 may be formed as an integral part of the rest of the pusher 37 or as a separate element which connects with the rest of the pusher 37. The distal end of the insertion tube 22 is connected to second ends of the flexible strips 55, typically via the coupling member of the distal end 33. The pusher 37 is generally controlled via the manipulator 32 to deploy the assembly 35 and change an ellipticity of the assembly 35 according to the longitudinal displacement of the pusher 37 with respect to the insertion tube 22.

The actual basket assembly 35 structure may vary. For example, flexible strips 55 may be made of a printed circuit board (PCB), or of a shape-memory alloy.

Embodiments described herein refer mainly to a basket distal-end assembly 35, purely by way of example. In alternative embodiments, the disclosed techniques can be used with a catheter having a balloon-based distal-end assembly or of any other suitable type of distal-end assembly.

Catheter 40 is inserted in a folded configuration, through sheath 23, and only after the catheter 40 exits sheath 23 is catheter 40 able to change shape by retracting pusher 37. By containing catheter 40 in a folded configuration, sheath 23 also serves to minimize vascular trauma on its way to the target location.

The distal end 33 of the catheter 40 comprises magnetic coil sensors 50A and 50B. The magnetic coil sensor 50A is shown in inset 45 at the distal edge of insertion tube 22 (i.e., at the proximal edge of basket assembly 35). The sensor 50A may be a Single-Axis Sensor (SAS), or a DAS or a TAS. Similarly, the sensor 50B may be a SAS, DAS, or TAS. Magnetic coil sensors 50A and 50B and electrodes 48 are connected by wires running through insertion tube 22 to various driver circuitries in a console 24.

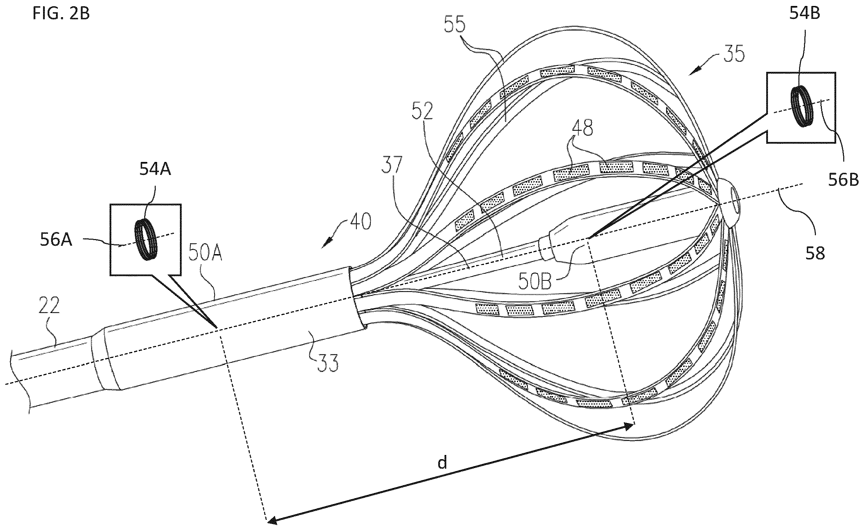

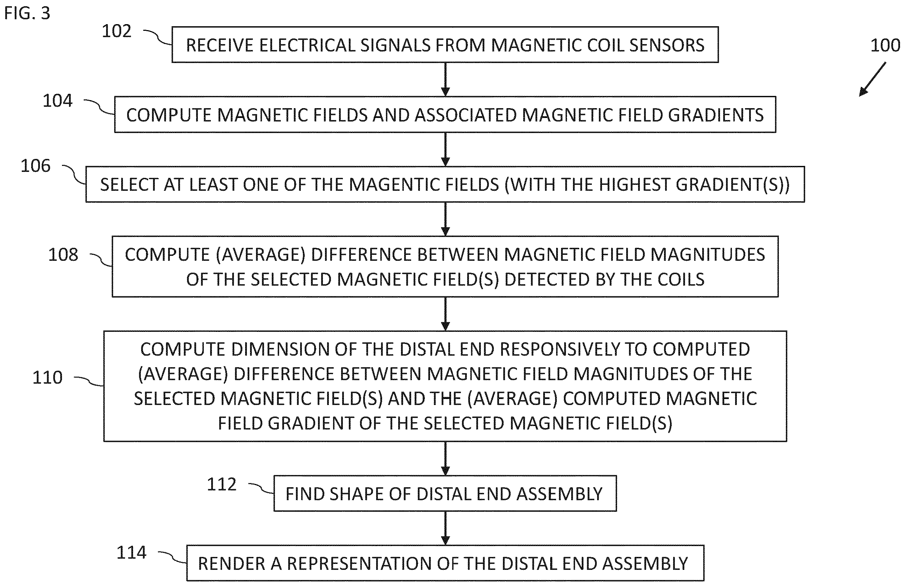

In some embodiments, system 20 comprises a magnetic-sensing subsystem to estimate an ellipticity of the basket assembly 35 of catheter 40, as well as its elongation/retraction state, inside a cardiac chamber of heart 26 by estimating the elongation of the basket assembly 35 from the distance between sensors 50A and 50B as described in more detail with reference to Figs. 2B and 3. Patient 28 is placed in a magnetic field generated by a pad containing multiple magnetic field generator coils 42, which are driven by a unit 43. The magnetic field generator coils 42 are configured to generate respective alternating magnetic fields, having respective different frequencies, into a region where a body-part (e.g., the heart 26) of a living subject (e.g., the patient 28) is located. The magnetic coil sensors 50A and 50B are configured to output electrical signals as a function of detecting the respective magnetic fields. For example, if there are nine magnetic field generator coils 42 generating nine respective different alternating magnetic fields with nine respective different frequencies, the electrical signals output by the magnetic coil sensors 50 will include components of the nine different frequency alternating magnetic fields. The magnitude of each of the magnetic fields varies with distance from the respective magnetic field generator coils 42 such that the location of the magnetic coil sensors 50 may be determined from the magnetic fields sensed by the magnetic coil sensors 50. Therefore, the transmitted alternating magnetic fields generate the electrical signals in sensors 50A and 50B, so that the electrical signals are indicative of position and orientation of the magnetic coil sensors 50. The magnetic coil sensors 50A and 50B are described in more detail with reference to Fig. 2B.

The generated signals are transmitted to console 24 and become corresponding electrical inputs to processing circuitry 41. The processing circuitry 41 may use the signals to compute: the elongation of the basket assembly 35, in order to estimate basket ellipticity and elongation/retraction state from the calculated distance between sensors 50A and 50B, described in more detail below with reference to Figs. 2B and 3; and compute a relative orientation between the axes of the sensors 50A and 50B to estimate a shape of the expandable distal end assembly 35 (e.g., a basket shape) based on the relative orientation, as described in more detail below.

The bow of the flexible strips 55 and/or the positions of the electrodes 48 (or other features) on the flexible strips 55 with respect to a fixed point on the catheter 44 (such as the distal tip of the insertion tube 22) may be measured for various distances between the magnetic sensors 50A, 50B and for various relative orientation angles between the magnetic sensors 50A, 50B. For example, the positions of the electrodes 48 with respect to the fixed point on the catheter 40 may be measured for every 0.2 mm movement of the pusher 37 with respect to the insertion tube 22 and for every 1 degree of relative orientation between the magnetic sensors 50A, 50B (up to a maximum sideways movement of the assembly 35). At each different distance/relative-orientation combination, the computed distance and computed relative orientation angle between the magnetic sensors 50A, 50B is recorded along with the position data of the electrodes 48. This data may then be used to estimate the bow of the flexible strips 55 and/or the positions of the electrodes 48 (or other features) on the flexible strips 55 with respect to a fixed point on the catheter 40 (such as the distal tip of the insertion tube 22) based on the computed distance and relative orientation angle between the magnetic sensors 50A, 50B.

Additionally, or alternatively, the bow of the flexible strips 55 may be estimated based on the following assumptions: (a) each of the flexible strips 55 is of a fixed and known length; (b) each of the flexible strips 55 is connected to the pusher 37 via a coupler, with the distal ends of the flexible strips 55 being substantially perpendicular (within an error of plus or minus 10 degrees) to the longitudinal axis 58; (c) each of the flexible strips 55 is connected to the insertion tube 22 via a coupler, which couples the proximal ends of the flexible strips 55 to the insertion tube 22, substantially parallel (within an error of plus or minus 10 degrees) to the longitudinal axis 58 of the insertion tube 22. Based on the above assumptions (a)-(c), and the computed positions of the couplers based on the computed positions of the magnetic sensors 50A, 50B, the bow of each of the flexible strips 55 may be computed using a third-degree polynomial. In some embodiments, the bow of the flexible strips 55 and/or the positions of the electrodes 48 (or other features) on the flexible strips 55 with respect to a fixed point on the catheter 40 (such as the distal tip of the insertion tube 22) may be computed based on the computed distance and orientation between the magnetic sensors 50A, 50B and a model of the catheter 40 which provides the bow of the flexible strips 55 and/or the positions of the electrodes 48 for the computed distance based on the mechanical properties and dimensions of the flexible strips 55.

A method of position and/or direction sensing using external magnetic fields and magnetic coil sensors, such as sensors 50A and 50B, is implemented in various medical applications, for example, in the CARTO^{®} system, produced by Biosense-Webster, and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1.

Processing circuitry 41, typically part of a general-purpose computer, is further connected via a suitable front end and interface circuits 44, to receive signals from body surface-electrodes 49. Processing circuitry 41 is connected to surface-electrodes 49 by wires running through a cable 39 to the chest of patient 28. The catheter 40 includes a connector 47 disposed at the proximal end 29 of the insertion tube 22 for coupling to the processing circuitry 41.

In some embodiments, processing circuitry 41 renders to a display 27, a representation 31 of at least a part of the catheter 40 and a body-part, (e.g., from a mapping process or from a scan (e.g., CT or MRI) of the body-part previously registered with the system 20), based on computed position coordinates of the insertion tube 22 and the flexible strips 55, described in more detail with reference to Fig. 3.

Processing circuitry 41 is typically programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

The example illustration shown in Fig. 1 is chosen purely for the sake of conceptual clarity. Fig. 1 shows only elements related to the disclosed techniques for the sake of simplicity and clarity. System 20 typically comprises additional modules and elements that are not directly related to the disclosed techniques, and thus are intentionally omitted from Fig. 1 and from the corresponding description. The elements of system 20 and the methods described herein may be further applied, for example, to control an ablation of tissue of heart 26.

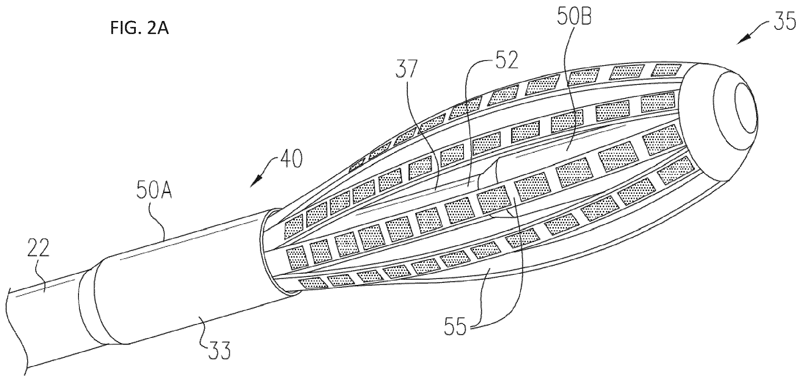

Reference is now made to Figs. 2A and 2B. Fig. 2A is a schematic view of a distal end 33 of the basket catheter 40 in a collapsed formation. Fig. 2B is a schematic view of the distal end 33 of the basket catheter 40 of Fig. 2A in a deployed formation.

The assembly 35 is typically an expandable distal end assembly (e.g., basket distal end assembly) comprising the flexible strips 55 (only some labeled for the sake of simplicity) disposed circumferentially around a distal portion 52 of the pusher 37 with first ends of the strips 55 connected to the distal end 33 (e.g., the coupling member of the distal end 33) of the insertion tube 22 and second ends of the strips 55 connected to the distal portion 52 (e.g., the coupling member of the distal portion 52) of the pusher 37. The flexible strips 55 are configured to bow radially outward when the pusher 37 is retracted. A plurality of the electrodes 48 (only some labeled for the sake of simplicity) are disposed on each of the flexible strips 55.

The magnetic coil sensor 50A is a coil-based position sensor disposed at the distal end 33 of the insertion tube 22, for example, in the coupling member at the distal end 33. The magnetic coil sensor 50A includes a coil 54A having an axis 56A. The magnetic coil sensor 50B is a coil-based position sensor disposed on the distal portion 52 of the pusher 37, for example, in a coupling member of the distal portion 52, coupling the distal ends of the flexible strips 55 to pusher 37. The magnetic coil sensor 50B includes a coil 54B having an axis 56B. The distal end 33 of the catheter 40 has a longitudinal axis 58. The magnetic coil sensors 50A, 50B are disposed on the distal end 33 with the axis 56A being substantially parallel with the axis 56B. In some embodiments, the axis 56A, the axis 56B, and the longitudinal axis 58 are substantially coaxial.

The pusher 37 is configured to be advanced and retracted through the insertion tube 22. The magnetic field sensors 50A, 50B are configured to move with respect to each other along the longitudinal axis 58 of the catheter 40 as the expandable distal end assembly 35 is expanded and collapsed. When the expandable distal end assembly 35 is collapsed a distance, d, between the magnetic coil sensors 50A, 50B increases, and when the expandable distal end assembly 35 is deployed (i.e. expanded) the distance, d, between the magnetic coil sensors 50A, 50B decreases.

Each sensor 50A, 50B may be a SAS, DAS or TAS. The sensors 50A, 50B may be the same type of sensor, or different types of sensors. If both of the sensors 50A, 50B are single-axis sensors, the catheter 40 generally includes another position sensor to track a roll of the assembly 35.

Reference is now made to Fig. 3, which is a flowchart 100 including steps in a method of operation of the system 20 of Fig. 1. Reference is also made to Fig. 2B.

As previously mentioned, the magnetic coil sensors 50A and 50B are configured to output electrical signals due to the inductive effect of each coil in response the respective magnetic fields. For example, if there are nine magnetic field generator coils 42 generating nine respective different alternating magnetic fields with nine respective different frequencies, the electrical signals output by the magnetic coil sensors 50 will include components of the nine respective different frequency alternating magnetic fields. The magnitude of each of the magnetic fields varies with distance from the respective magnetic field generator coils 42 such that the location of the magnetic coil sensors 50 may be determined from the magnetic fields sensed by the magnetic coil sensors 50. Therefore, the transmitted alternating magnetic fields generate electrical signals in sensors 50A and 50B, such that the electrical signals are indicative of positions and orientation of the magnetic coil sensors 50. The processing circuitry 41 is configured to receive (block 102) the electrical signals from the magnetic coil sensors 50A, 50B.

The processing circuitry 41 is configured to compute (block 104) the magnetic fields detected by the magnetic coil sensor 50A and the magnetic coil sensor 50B, and respective magnetic field gradients (e.g., parallel to the direction of the axes 56A, 56B of the coils 54A, 54B) of respective ones of the magnetic fields detected at the distal end 33. That is, the processing circuitry 41 computes the magnetic field and associated magnetic field gradient from at least one of the electrical signals received by the circuitry 41 from one of more of the magnetic coil sensors 54A and 54B. In some embodiments, an approximate position (location and orientation) of one or more of the magnetic field sensors 50A, 50B may be computed using any suitable method and then based on a known function of the different magnetic fields over three-dimensional (3D) space, the magnetic field gradients at the distal end 33 (e.g., parallel to the direction of the axes 56A, 56B of the coils 54A, 54B) may be found for each of the magnetic fields. The position of the distal end 33 may be computed based on an average position of the magnetic coil sensors 50A, 50B or based on the most accurate sensor of the magnetic coil sensors 50A, 50B. For example, if the sensor 50B is a DAS or TAS, then the location and orientation of sensor 50B may be computed based on all or some of the sensing coils of that sensor.

The processing circuitry 41 is configured to select (block 106) one of magnetic fields having a respective one of the computed magnetic field gradients. In some embodiments, the selected magnetic field has a highest computed magnetic field gradient of the computed magnetic field gradients (i.e. the magnetic field with the highest gradient is selected). The highest magnetic field gradient generally indicates that the selected magnetic field will provide the highest sensitivity in the direction parallel to the axes 56A, 56B of the coils 54A, 54B of the magnetic coil sensors 50A, 50B and will therefore provide the highest accuracy in computing the distance, d, between the sensors 50A, 50B.

In some embodiments, the processing circuitry 41 is configured to select a subset of the magnetic fields (e.g., having the highest magnetic field gradients among the magnetic fields) and compute an average magnetic field gradient of the selected magnetic fields. Therefore, the processing circuitry 41 is configured to select at least one of the magnetic fields having a magnetic field gradient (e.g., an average computed magnetic field gradient) as a function of at least one of the electrical signals received by the circuitry 41 from the coils (which are used to compute the magnetic fields and the magnetic field gradients of the respective magnetic fields).The processing circuitry 41 is configured to compute (block 108) a difference between magnetic field magnitudes of the selected magnetic field (e.g., the magnetic field with the highest gradient) detected by the magnetic coil sensor 50A and the magnetic coil sensor 50B. For example, if the magnetic field magnitude of the selected magnetic field detected by the magnetic coil sensor 50A is equal to B1 and the magnetic field magnitude of the selected magnetic field detected by the magnetic coil sensor 50B is equal to B2, the difference magnetic field magnitudes of the selected magnetic field (e.g., the magnetic field with the highest gradient) detected by the magnetic coil sensor 50A and the magnetic coil sensor 50B is equal to B2 minus B1.

In some embodiments, when a subset of magnetic fields are selected, the processing circuitry 41 is configured to compute a difference (which is an average difference) between magnetic field magnitudes of the selected magnetic fields (e.g., the magnetic field with the highest gradients) detected by the magnetic coil sensor 50A and the magnetic coil sensor 50B. For example, if the average magnetic field magnitude of the selected magnetic fields detected by the magnetic coil sensor 50A is equal to B3 and the average magnetic field magnitude of the selected magnetic fields detected by the magnetic coil sensor 50B is equal to B4, the average difference magnetic field magnitudes of the selected magnetic fields (e.g., the magnetic fields with the highest gradient) detected by the magnetic coil sensor 50A and the magnetic coil sensor 50B is equal to B4 minus B3.

The processing circuitry 41 is configured to compute (block 110) a dimension of the distal end 33, which is a function of the distance d between the magnetic coil sensors 50A and 50B, as a function of the computed difference (e.g., average difference) between the magnetic field magnitudes of the selected magnetic field(s) (e.g., B2 minus B1 or B4 minus B3) and the respective computed magnetic field (average) gradient (e.g., highest computed magnetic field gradient(s)) of the selected magnetic field(s). In some embodiments, the processing circuitry 41 is configured to compute the dimension of the distal end 33 based on the computed (average) difference between the magnetic field magnitudes of the selected magnetic field(s) (e.g., B2 minus B1 or B4 minus B3) divided by the respective computed (average) magnetic field gradient (e.g., highest computed magnetic field gradient(s)) of the selected magnetic field(s). The computed dimension may be the distance between the magnetic coil sensors 50A, 50B. In some embodiments, the computed dimension is a dimension of a shape of the distal end 33 of the catheter, for example, the distance between the proximal and distal points of the distal end assembly 35 or a circumference at the equator of the assembly 35. The processing circuitry 41 is configured to find (block 112) (e.g., by computation or from a lookup table) a shape of the distal end assembly 35 as a derivation from the computed dimension. The processing circuitry 41 is optionally configured to compute a relative orientation between the axes of the sensors 50A and 50B to estimate or derive the shape of the expandable distal end assembly 35 (e.g., a basket shape) based on the relative orientation. The processing circuitry 41 is configured to render (block 114) to the display 27 the representation 31 (Fig. 1) of the distal end assembly 35 as a derivation of the found shape of the distal end assembly 35. One technique for deriving a shape of the expandable distal end assembly 35 based on the distances between magnetic location sensors can be found in US Patent Application S.N. 16/854538 (Attorney Docket No. BIO6130USNP) filed April 21, 2020.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 72% to 108%.

## Claims

1. A medical system, comprising:
generator coils (42) configured to generate respective magnetic fields having respective different frequencies in a region of a body part of a living subject;
a catheter (40) configured to be inserted into the body part of the living subject, and comprising a longitudinal axis and a distal end, wherein the distal end includes an expandable distal end assembly (35) and includes magnetic coil sensors (50A, 50B) configured to output electrical signals in response to the respective magnetic fields, and including a first magnetic coil sensor (50A) having a first axis (56A) and a second magnetic coil sensor (50B) having a second axis (56B), the magnetic coil sensors (50A, 50B) being disposed on the distal end with the first axis (56A) being substantially parallel with the second axis (56B), wherein the first and second magnetic field sensors (50A, 50B) are configured to move with respect to each other along the longitudinal axis of the catheter (40) as the expandable distal end assembly is expanded and collapsed, wherein when the expandable distal end assembly (35) is collapsed the distance between the magnetic coil sensors (50A, 50B) increases and when the expandable distal end assembly (35) is deployed the distance between the magnetic coil sensors (50A, 50B) decreases; and
processing circuitry configured to:
receive the electrical signals from the magnetic coil sensors (50A, 50B);
select at least one of the magnetic fields having a magnetic field gradient defined by at least one of the received electrical signals;
compute a difference between magnetic field magnitudes of the at least one selected magnetic field detected by the first magnetic coil sensor (50A) and the second magnetic coil sensor (50B) based on the received electrical signals; and
compute a dimension of the distal end, which is a function of a distance between the magnetic coil sensors, based on the computed difference between the magnetic field magnitudes of the at least one selected magnetic field and the magnetic field gradient of the at least one selected magnetic field.

2. The system according to claim 1, wherein the dimension is the distance between the magnetic coil sensors (50A, 50B).

3. The system according to claim 1, wherein the dimension is a dimension of a shape of the distal end of the catheter.

4. The system according to claim 1, wherein the processing circuitry is configured to compute the dimension of the distal end as a function of the computed difference between the magnetic field magnitudes of the at least one selected magnetic field divided by the magnetic field gradient of the at least one selected magnetic field.

5. The system according to claim 1, wherein:
the at least one selected magnetic field includes one of the magnetic fields having a highest magnetic field gradient of the magnetic fields; and
the processing circuitry is configured to compute the dimension of the distal end as a function of the computed difference between the magnetic field magnitudes of the at least one selected magnetic field and the highest magnetic field gradient.

6. The system according to claim 1, wherein the first axis (56A), second axis (56B), and the longitudinal axis are substantially coaxial.

7. The system according to claim 1, wherein the expandable distal end assembly (35) is a basket distal end assembly comprising a plurality of flexible strips (55) and electrodes (48) disposed on the flexible strips.

8. The system according to claim 1, further comprising a display, and wherein the processing circuitry is configured to:
find a shape of the distal end assembly based on at least the computed dimension; and
render to the display a representation of the distal end assembly based on the found shape of the distal end assembly.

9. The system according to claim 8, wherein the computed dimension is the distance between the magnetic coil sensors (50A, 50B).

10. The system according to claim 1, wherein the processing circuitry is configured to:
compute a relative orientation between the first axis (56A) of the first magnetic coil sensor (50A) and the second axis (56B) of the second magnetic coil sensor (50B); and
estimate a shape of the distal end assembly based on the computed relative orientation.

## Patentansprüche

1. Medizinisches System, umfassend:
Generatorspulen (42), die konfiguriert sind, um jeweilige Magnetfelder, die jeweils unterschiedliche Frequenzen aufweisen, in einer Region eines Körperteils eines lebenden Subjekts zu generieren;
einen Katheter (40), der konfiguriert ist, um in das Körperteil des lebenden Subjekts eingeführt zu werden, und umfassend eine Längsachse und ein distales Ende, wobei das distale Ende eine expandierbare distale Endanordnung (35) einschließt und Magnetspulensensoren (50A, 50B) einschließt, die konfiguriert sind, um als Reaktion auf die jeweiligen Magnetfelder elektrische Signale auszugeben, und einen ersten Magnetspulensensor (50A), der eine erste Achse (56A) aufweist, und einen zweiten Magnetspulensensor (50B), der eine zweite Achse (56B) aufweist, einschließt, wobei die Magnetspulensensoren (50A, 50B) an dem distalen Ende angeordnet sind, wobei die erste Achse (56A) im Wesentlichen parallel zu der zweiten Achse (56B) verläuft, wobei der erste und der zweite Magnetfeldsensor (50A, 50B) konfiguriert sind, um sich in Bezug aufeinander entlang der Längsachse des Katheters (40) zu bewegen, während die expandierbare distale Endanordnung expandiert und zusammengeklappt wird, wobei, wenn die expandierbare distale Endanordnung (35) zusammengeklappt ist, der Abstand zwischen den Magnetspulensensoren (50A, 50B) zunimmt, und wenn die expandierbare distale Endanordnung (35) entfaltet ist, der Abstand zwischen den Magnetspulensensoren (50A, 50B) abnimmt; und
eine Verarbeitungsschaltung, die konfiguriert ist zum:
Empfangen der elektrischen Signale von den Magnetspulensensoren (50A, 50B);
Auswählen mindestens eines der Magnetfelder, die einen Magnetfeldgradienten aufweisen, der durch mindestens eines der empfangenen elektrischen Signale definiert ist;
Berechnen eines Unterschieds zwischen Magnetfeldgrößen des mindestens einen ausgewählten Magnetfelds, das durch den ersten Magnetspulensensor (50A) und den zweiten Magnetspulensensor (50B) erfasst wird, basierend auf den empfangenen elektrischen Signalen; und
Berechnen einer Abmessung des distalen Endes, die von einem Abstand zwischen den Magnetspulensensoren abhängig ist, basierend auf dem berechneten Unterschied zwischen den Magnetfeldgrößen des mindestens einen ausgewählten Magnetfelds und dem Magnetfeldgradienten des mindestens einen ausgewählten Magnetfelds.

2. System nach Anspruch 1, wobei die Abmessung der Abstand zwischen den Magnetspulensensoren (50A, 50B) ist.

3. System nach Anspruch 1, wobei die Abmessung eine Abmessung einer Form des distalen Endes des Katheters ist.

4. System nach Anspruch 1, wobei die Verarbeitungsschaltung konfiguriert ist, um die Abmessung des distalen Endes in Abhängigkeit von dem berechneten Unterschied zwischen den Magnetfeldgrößen des mindestens einen ausgewählten Magnetfelds dividiert durch den Magnetfeldgradienten des mindestens einen ausgewählten Magnetfelds zu berechnen.

5. System nach Anspruch 1, wobei:
das mindestens eine ausgewählte Magnetfeld eines der Magnetfelder, die einen höchsten Magnetfeldgradienten aufweisen, der Magnetfelder einschließt; und
die Verarbeitungsschaltung konfiguriert ist, um die Abmessung des distalen Endes in Abhängigkeit von dem berechneten Unterschied zwischen den Magnetfeldstärken des mindestens einen ausgewählten Magnetfelds und dem höchsten Magnetfeldgradienten zu berechnen.

6. System nach Anspruch 1, wobei die erste Achse (56A), die zweite Achse (56B) und die Längsachse im Wesentlichen koaxial sind.

7. System nach Anspruch 1, wobei die expandierbare distale Endanordnung (35) eine distale Korbendanordnung ist, umfassend eine Vielzahl von flexiblen Streifen (55) und Elektroden (48), die auf den flexiblen Streifen angeordnet sind.

8. System nach Anspruch 1, ferner umfassend eine Anzeige, und wobei die Verarbeitungsschaltung konfiguriert ist zum:
Ermitteln einer Form der distalen Endanordnung basierend auf mindestens der berechneten Abmessung; und
Rendern auf der Anzeige eine Darstellung der distalen Endanordnung basierend auf der ermittelten Form der distalen Endanordnung.

9. System nach Anspruch 8, wobei die berechnete Abmessung der Abstand zwischen den Magnetspulensensoren (50A, 50B) ist.

10. System nach Anspruch 1, wobei die Verarbeitungsschaltung konfiguriert ist zum:
Berechnen einer relativen Ausrichtung zwischen der ersten Achse (56A) des ersten Magnetspulensensors (50A) und der zweiten Achse (56B) des zweiten Magnetspulensensors (50B); und
Schätzen einer Form der distalen Endanordnung basierend auf der berechneten relativen Ausrichtung.

## Revendications

1. Système médical, comprenant :
des bobines génératrices (42) configurées pour générer des champs magnétiques respectifs ayant des fréquences différentes respectives dans une région d'une partie du corps d'un sujet vivant ;
un cathéter (40) configuré pour être inséré dans la partie du corps du sujet vivant, et comprenant un axe longitudinal et une extrémité distale, dans lequel l'extrémité distale comporte un ensemble (35) extrémité distale expansible et comporte des capteurs à bobine magnétique (50A, 50B) configurés pour délivrer en sortie des signaux électriques en réponse aux champs magnétiques respectifs, et comportant un premier capteur à bobine magnétique (50A) ayant un premier axe (56A) et un second capteur à bobine magnétique (50B) ayant un second axe (56B), les capteurs à bobine magnétique (50A, 50B) étant disposés sur l'extrémité distale, le premier axe (56A) étant sensiblement parallèle au second axe (56B), dans lequel les premier et second capteurs de champ magnétique (50A, 50B) sont configurés pour se déplacer l'un par rapport à l'autre le long de l'axe longitudinal du cathéter (40) à mesure que l'ensemble extrémité distale expansible est expansé et replié, dans lequel, lorsque l'ensemble (35) extrémité distale expansible est replié, la distance entre les capteurs à bobine magnétique (50A, 50B) augmente et lorsque l'ensemble extrémité distale expansible (35) est déployé, la distance entre les capteurs à bobine magnétique (50A, 50B) diminue ; et
une circuiterie de traitement configurée pour :
recevoir les signaux électriques des capteurs à bobine magnétique (50A, 50B) ;
sélectionner au moins l'un parmi les champs magnétiques ayant un gradient de champ magnétique défini par au moins l'un parmi les signaux électriques reçus ;
calculer une différence entre des intensités de champ magnétique de l'au moins un champ magnétique sélectionné détecté par le premier capteur à bobine magnétique (50A) et le second capteur à bobine magnétique (50B) sur la base des signaux électriques reçus ; et
calculer une dimension de l'extrémité distale, qui est une fonction d'une distance entre les capteurs à bobine magnétique, sur la base de la différence calculée entre les intensités de champ magnétique de l'au moins un champ magnétique sélectionné et le gradient de champ magnétique de l'au moins un champ magnétique sélectionné.

2. Système selon la revendication 1, dans lequel la dimension est la distance entre les capteurs à bobine magnétique (50A, 50B).

3. Système selon la revendication 1, dans lequel la dimension est une dimension d'une forme de l'extrémité distale du cathéter.

4. Système selon la revendication 1, dans lequel la circuiterie de traitement est configurée pour calculer la dimension de l'extrémité distale en fonction de la différence calculée entre les intensités de champ magnétique de l'au moins un champ magnétique sélectionné divisée par le gradient de champ magnétique de l'au moins un champ magnétique sélectionné.

5. Système selon la revendication 1, dans lequel :
l'au moins un champ magnétique sélectionné comporte l'un des champs magnétiques ayant un gradient de champ magnétique le plus élevé parmi les champs magnétiques ; et
la circuiterie de traitement est configurée pour calculer la dimension de l'extrémité distale en fonction de la différence calculée entre les intensités de champ magnétique de l'au moins un champ magnétique sélectionné et le gradient de champ magnétique le plus élevé.

6. Système selon la revendication 1, dans lequel le premier axe (56A), le second axe (56B) et l'axe longitudinal sont sensiblement coaxiaux.

7. Système selon la revendication 1, dans lequel l'ensemble (35) extrémité distale expansible est un ensemble extrémité distale en panier comprenant une pluralité de bandes flexibles (55) et d'électrodes (48) disposées sur les bandes flexibles.

8. Système selon la revendication 1, comprenant en outre un écran, et dans lequel la circuiterie de traitement est configurée pour :
trouver une forme de l'ensemble extrémité distale sur la base d'au moins la dimension calculée ; et
afficher sur l'écran une représentation de l'ensemble extrémité distale sur la base de la forme trouvée de l'ensemble extrémité distale.

9. Système selon la revendication 8, dans lequel la dimension calculée est la distance entre les capteurs à bobine magnétique (50A, 50B).

10. Système selon la revendication 1, dans lequel la circuiterie de traitement est configurée pour :
calculer une orientation relative entre le premier axe (56A) du premier capteur à bobine magnétique (50A) et le second axe (56B) du second capteur à bobine magnétique (50B) ; et
estimer une forme de l'ensemble extrémité distale sur la base de l'orientation relative calculée.
